# EUROPEAN PATENT APPLICATION

(11) **EP 1 400 807 A2**
(43) Date of publication of application: **24.03.2004**
(21) Application number: 02078917.8
(22) Date of filing: 17.09.2002
(51) Int. Cl.: G01N 33/50, C12N 5/06

(54) **Use of novel stem cell markers for isolation of intestinal stem cells, and use of the intestinal stem cells thus obtained for the preparation of a therapeutical composition**

(71) Applicant: Kylix B.V., 3971 JD Driebergen (NL)
(72) Inventor: Clevers, Johannes Carolus, 3712 AP Huis ter Heide (NL); van de Wetering, Marcus Lambertus, 3994 TD Houten (NL); Gomez, Eduard Batlle, 3582 JD Utrecht (NL); Sancho Suils, Elena, 3582 JD Utrecht (NL)
(74) Representative: Van Someren, Petronella F. H. M.

(57) **Abstract**

The invention relates to the use of the expression products of the TCF target genes as markers for the isolation of intestinal stem cells in a method for the isolation of intestinal stem cells by providing antibodies, antibody derivatives or ligand fusion proteins against one or more of the target gene products of the genes; providing single cell suspensions of intestinal tissue; contacting the antibodies, antibody derivatives or ligand fusion proteins and the single cell suspensions; identifying the cells binding to the antibodies, antibody derivatives or ligand fusion proteins; and optionally isolating the stem cells from the antibodies, antibody derivatives or ligand fusion proteins.

## Description

The present invention relates to the use of novel stem cell markers for the isolation of intestinal stem cells. The invention further relates to the use of stem cells thus obtained for the preparation of a therapeutical composition for treatment of damaged and diseased tissues, particularly those of endodermal origin such as liver, pancreas and intestine.

Turnover of the epithelial cell lineages within the gastrointestinal tract is a constant process, occurring every 2-7 days under normal homeostasis and increasing after damage. This process is regulated by multipotent stem cells, which give rise to all gastrointestinal epithelial cell lineages and can regenerate whole intestinal crypts and gastric glands. The stem cells of the gastrointestinal tract are as yet undefined. Consequently, there is a lack of structural markers specific to the stem cell compartment.

The processes of cell renewal, lineage commitment and cell differentiation are intimately coupled to cell migration in a precise and spatially organized manner. At the bottom of the crypts, the intestinal stem cells give rise to a transient population of undifferentiated cells that vigorously proliferate as they migrate towards the lumen of the intestine. These undifferentiated precursors commit to different cell lineages during their migration and undergo cell cycle arrest and differentiation upon reaching the top of the crypts. This basic pattern of cell behavior is conserved in the small intestine, with one significant difference, namely the precursors follow a bi-directional migratory path. Goblet, enteroendocrine and absorptive cells migrate towards the lumen of the gut, but a specialized group of cells called the Paneth cells migrates towards the bottom of the crypt.

It is not known how the directional migration is controlled or how the different cell compartments within the intestinal epithelium are established.

Since intestinal stem cells are potentially useful in the repair of damaged epithelium, liver tissue or other non-endodermal tissues, there is a clear need for a method that enables the isolation of intestinal stem cells. It is therefore the object of the present invention to provide markers that distinguish stem cells from other cells residing in the gastrointestinal tract.

The present inventors have now found that the expression of EpHB2 and EpHB3 tyrosine kinase receptors in colorectal cancer cells is dependent upon active ß-catenin/ TCF signalling, whereas the expression of their ligand ephrin-B1 is induced upon inhibition of ß-catenin/TCF activity. Accordingly, proliferative cells that reside in the intestinal crypts express both Eph receptors, whereas ephrin-B ligands are expressed in a complementary fashion on the adjacent differentiated cells.

It was thus found that the genetic program which defines the phenotype of the stem cells in the gut is controlled by TCF/ß-catenin.

The TCF target genes listed in Table 1 collectively define the stem cell phenotype of the gut. A number of these target genes (listed in Table 2) encode cell-surface proteins. The present inventors contemplated that these can

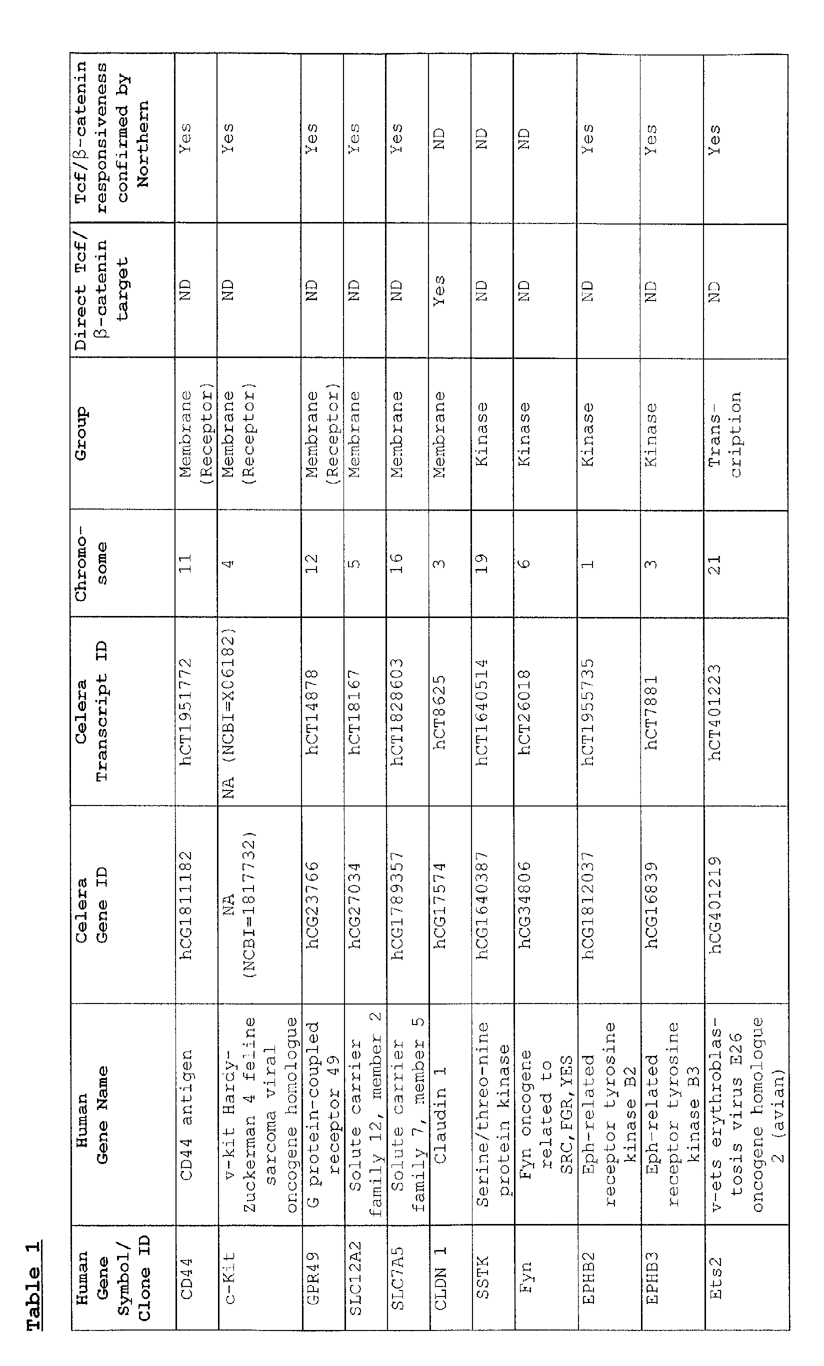

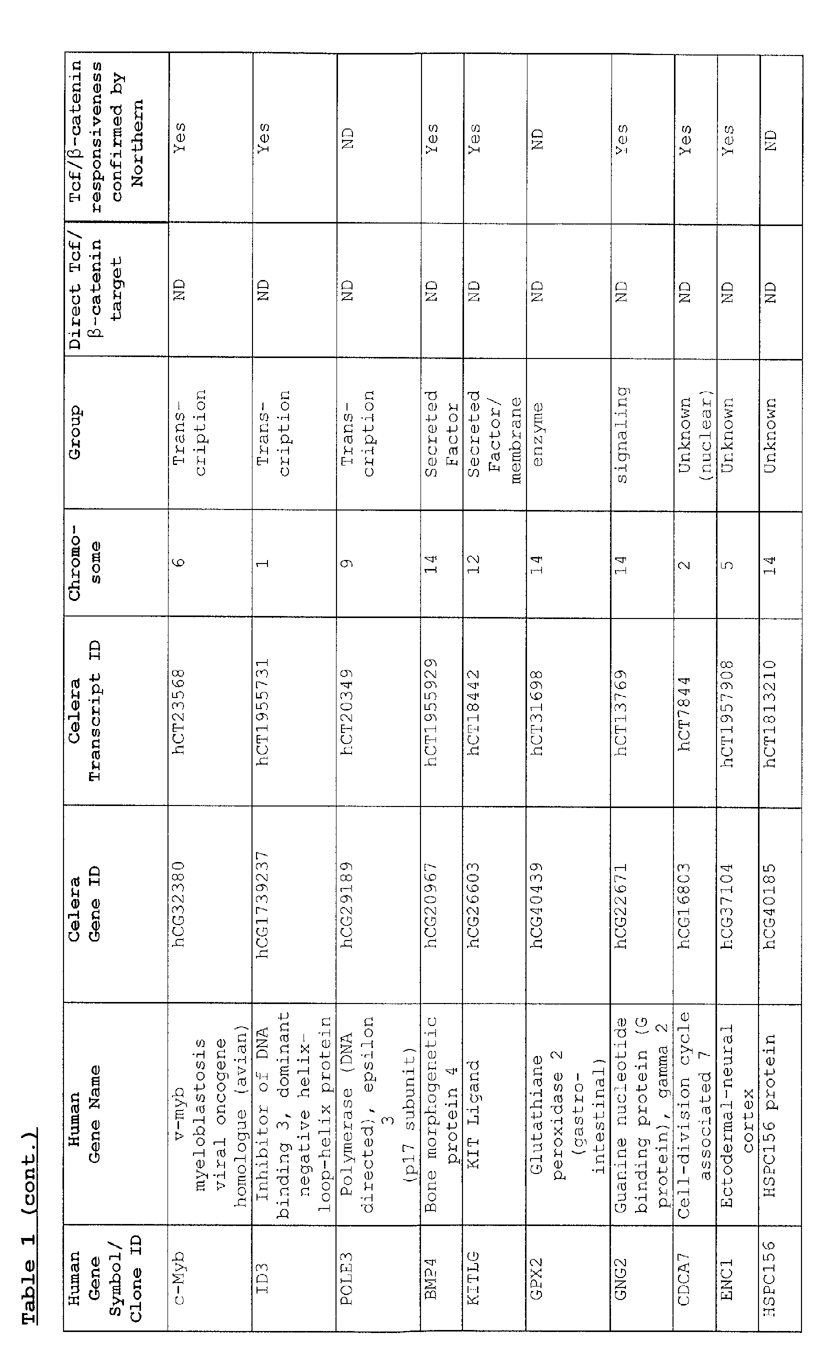

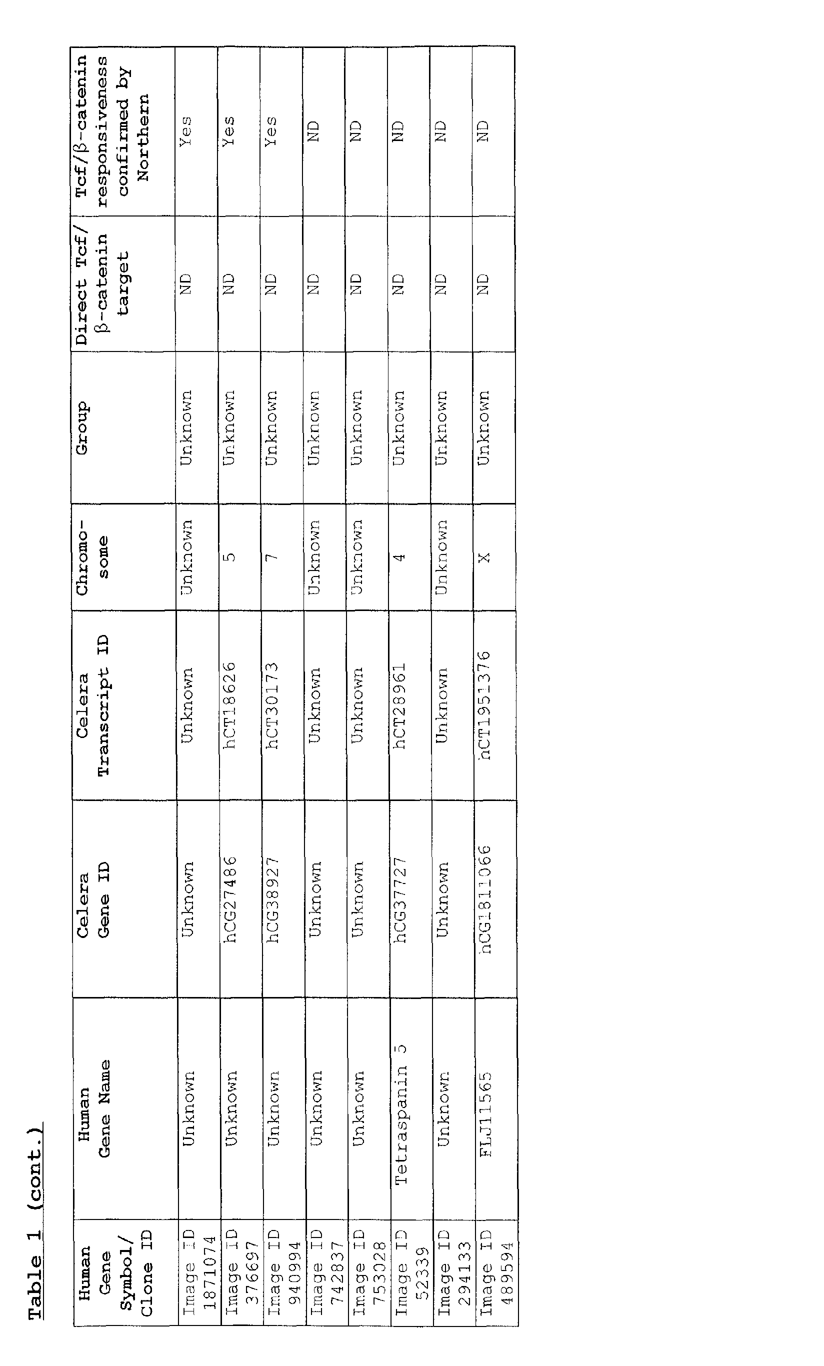

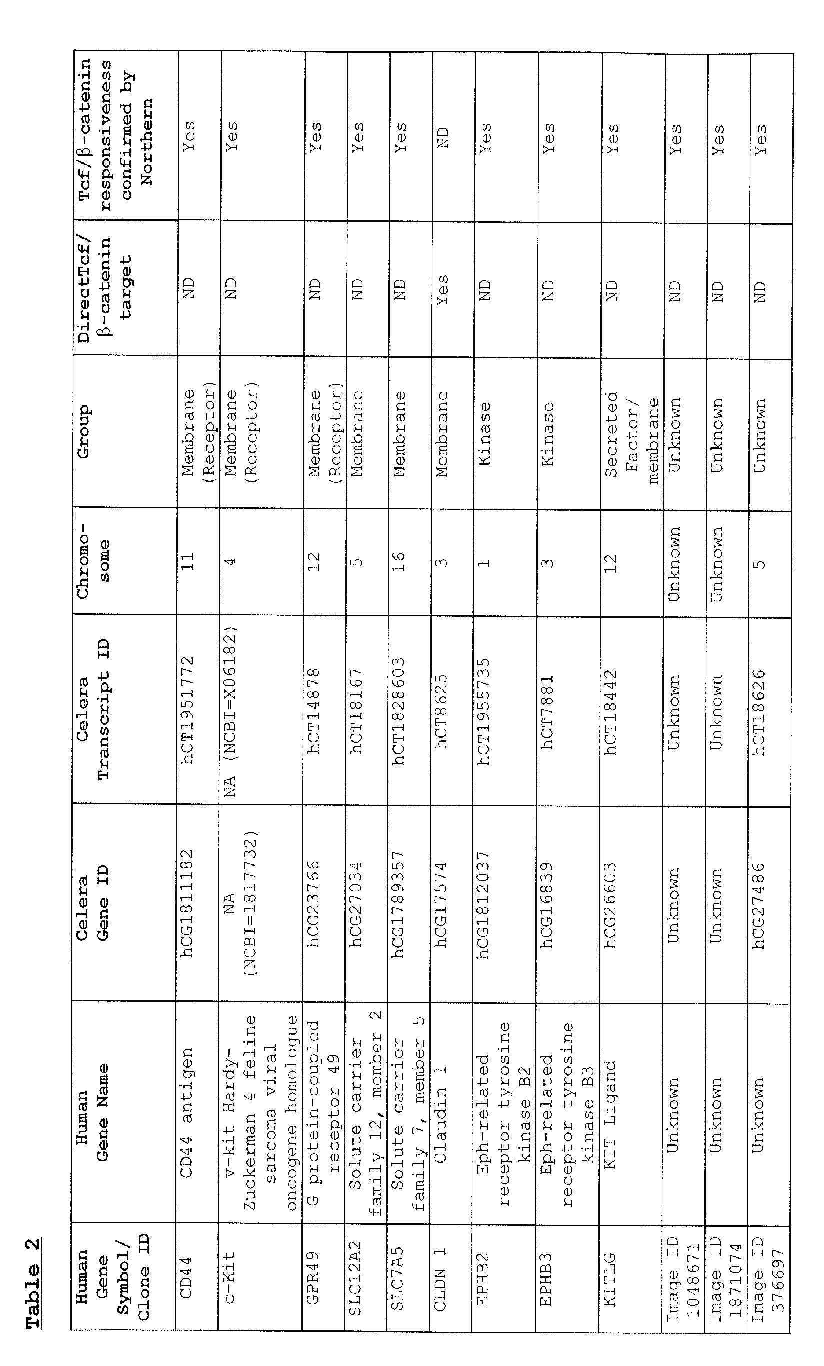

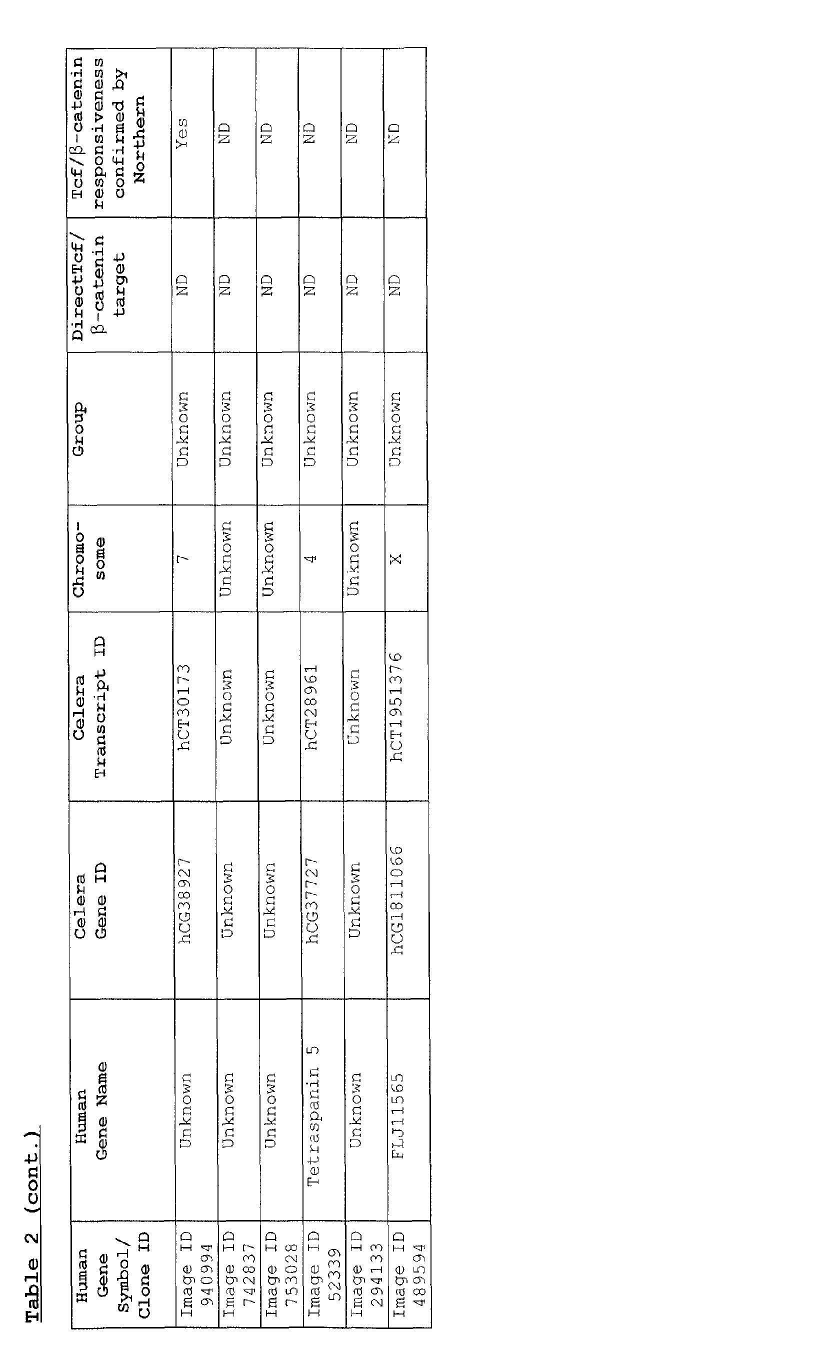

be used as markers for selection of the abundant stem cell population in the gut. Subsequently, such isolated stem cells can be used for tissue replacement and/or treatment of various diseases.

The present invention thus relates to the use of the TCF target gene products listed in Table 2 for isolating intestinal stem cells.

According to another aspect of the invention the use is provided of intestinal stem cells for the preparation of a therapeutical composition for the treatment of various diseases in which organs of endodermal origin, in particular the liver, pancreas and/or the intestinal epithelium are diseased or damaged, such as in Type 1 diabetes. The stem cells may be genetically modified to express (poly)peptides that can help in treating particular diseases of the liver (such as liver damage caused by alcohol), pancreas or intestine.

The TCF target gene products are used for isolating intestinal stem cells by the following steps:
a) providing antibodies, antibody derivatives or ligand fusion proteins against one or more of the target gene products of the genes listed in Table 2;
b) providing single cell suspensions of intestinal tissue;
c) contacting the antibodies, antibody derivatives or ligand fusion proteins and the single cell suspensions;
d) identifying the cells binding to the antibodies, antibody derivatives or ligand fusion proteins; and
e) optionally isolating the stem cells from the antibodies, antibody derivatives or ligand fusion proteins.

More in particular, a FACS cell sorter can be used to identify and sort the cells that bind the antibody or derivatives thereof. Cells may be distinguished by the presence or absence of a particular marker, but also by the degree of expression of one or more particular markers, but also by the degree of expression of one or more particular markers. Preferably, stem cells are sorted based on the fact that they coexpress high levels of CD44 and EpHB2 and low levels of EpHB3. Sorted stem cell populations should also demonstrate only low level EFNB1 expression. Suitable combinations of other stem cell markers can be used in the same manner.

Antibodies or their derivatives can be provided by methods that are well known to the person skilled in the art (such methods include, but are not limited to the hybridoma technique as described by Kohler and Milstein in Nature 256:494-497, 1975 & Cambell in "Monoclonal antibody technology, the production and characterization of rodent and human hybridomas" in Burdon et al., EDS, Laboratory Techniques in Biochemistry and Molecular Biology Vol 13, Elsevier Science Publishers, Amsterdam (1985)) or can be produced by means of transgenic mice carrying a minichromosome which enables the generation of fully human monoclonal antibodies following immunisation with the target protein, or may be commercially available.

Ligand fusion proteins, such as ephrin:Fc fusion proteins, can be made by means of well-known genetic engineering techniques.

Single cell suspensions that survive more than 5 to 10 days of culturing can be made by adapting well known methods such as those disclosed in Cano-Gauci, D et al., (1993) Exp. Cell Res. 208: 344, Booth et al., (1995) Epithelial Cell Biol 4: 76 or Booth, C et al., (1999) Exp. Cell Res. 249: 359). The method of Booth et al., 1999 is a good starting point, with cultures of crypt cell preparations being supplemented with Wnt conditioned media to maintain the pluripotentency of the stem cells.

Alternatively, it may not be necessary to culture the cells for more than a couple of days if the stem cells are immediately purified by FACS sorting and cryopreserved or prepared for immediate application.

The tissue used for the preparation of single cell suspensions is preferably human tissue.

Cells binding to antibodies or antibody derivatives are preferably identified by means of a FACS sorter. However, alternative techniques are also encompassed in this invention. Examples of such techniques are magnetic separation with antibody-coated magnetic beads, affinity chromatography and so-called panning with antibody attached to a solid matrix.

Isolating the cells from the antibodies is not always necessary but can be done in various manners well known to the person skilled in the art and including enzymatic digestion of any spacer sequences between antibody and any solid phase, mechanical agitation or other appropriate methods.

The stem cells isolated according to the invention can be used as a therapeutic agent for the treatment of diseases in which intestinal epithelium cells are affected. Example thereof are inflammatory conditions such as Crohn's disease and tissue damage resulting from chemotherapy or other toxic agents. In addition, the stem cells can be used to repair diseased pancreas, for example in type 1 diabetes, or
cirrhosis of the liver (alcohol-induced damage).

When stem cells are used as therapeutic agent for treating the liver they are for example administered via the portal vein. In order to commit the cells to a particular lineage stem cells can be treated with one or more specific cytokines prior to administration.

The present invention is further illustrated in the Examples that follow. Example 1 shows how the genes that define the phenotype of the intestinal stem cells are identified. Example 2 shows how the information obtained in Example 1 can be used to isolate intestinal stem cells. Example 3 relates to the use of the stem cells thus obtained to prepare a therapeutical composition.

### EXAMPLES

### EXAMPLE 1

### Identification of target genes that define the phenotype of the intestinal stem cell

### INTRODUCTION

A comprehensive analysis of the genetic program controlled by the ß-catenin/TCF signalling pathway in intestinal epithelial cells was performed. Ls174T colorectal cancer cells show high levels of constitutive ß-catenin/TCF-driven transcription due to stabilizing mutations in ß-catenin. Ls174T cells that express dominant-negative TCF factors (DN-TCF) under the control of a doxycycline-inducible promoter were engineered. Upon induction with doxycycline, transcription mediated by ß-catenin/TCF complexes is abrogated in these cells. The gene expression profile of these cells was evaluated before and after the induction of DN-TCFs using a 24,000 cDNA array chip. This example gives an analysis of the changes in the expression profiles of these cells as well as an evaluation of the genetic program driven by ß-catenin/TCF in colorectal cancer. As a common denominator, it was found that genes downregulated by DN-TCF in colorectal cancer cell lines were physiologically expressed by the proliferative cells within the crypts. By contrast, genes that were upregulated upon the inhibition of ß-catenin/TCF were expressed in the differentiated compartments of the intestinal epithelium. The downregulated genes are thus markers for proliferating cells, such as stem cells.

### MATERIAL AND METHODS

### 1. Cell culture and transfections

Cells were grown in RPMI supplemented with 10% FCS and antibiotics. T-REx system (Invitrogen) was used according to manufacturer's instructions to generate inducible dnTCF inducible CRC cell lines. In short, 10 cells were transfected by electroporation with 20 µg FspI linearized pcDNA₆TR. After 3 weeks of selection, blasticidin (10 µg/ml) resistant colonies were expanded and transfected with pcDNA₄TO-Luciferase. From each cell line, two clones showing the strongest induction were chosen. These were subsequently transfected with 20 µg dnTCF4 in pCDNATO. After selection on Zeocin (500 µg/ml), resistant colonies were tested for dnTCF induction by immunocytochemical staining after addition of doxycycline and selected for further studies.

### 2. Cell cycle analysis

3 x 10⁶ (Ls174T) cells were seeded in 9 cm dishes and doxycycline was added (1 µg/ml). After 20 hrs, BrdU (Roche) was added for 20 min. Cells were then collected and fixed in ethanol 70%. Nuclei were isolated, incubated withα-BrdU-FITC (BD) and cell cycle profiles were determined by FACS analysis. Crystal violet staining on methanol fixed cells were done on cells after 5 days in culture with or without the addition of doxycycline.

### 3. RNA isolation

RNA was isolated using Trizol (Gibco). Probes were obtained by appropriate restriction enzyme digestion of corresponding IMAGE clones (IMAGE consortium) spotted in the array.

### 4. Immunohistochemistry

The antibodies against EPHB2 and EPHB3 were obtained from R&D systems and against ß-catenin from Transduction Laboratories. Immunostainings were performed according to standard procedures. Briefly, sections were pretreated with peroxidase blocking buffer (100 mM Na-phosphate pH 5.8, 30 mM NaN₃, 0.2% H₂O₂) for 20 minutes at room temperature after dewaxing and hydration. Antigen retrieval was performed by boiling samples in 10 mM Na-citrate buffer pH 6.0, for 20 minutes. For ß-catenin stainings, samples were boiled for 45 minutes in 40 mM Tris pH 8.0 containing 1 mM EDTA. Incubation of antibodies was performed in 1% BSA in PBS 1 hour at room temperature. In all cases, the Envision+ kit (DAKO) was used as a secondary reagent. Stainings were developed using DAB (brown precipitate). Slides were then counterstained with hematoxylin and mounted.

### 5. Probe preparation and microarray procedures

mRNA was extracted from cells using the Fasttrack 2.0 procedure (Invitrogen Inc.) following the manufacturer's directions. Fluorescent labeled cDNA was prepared from 1µg of polyA mRNA by oligo dT-primed polymerization using Superscript II reverse transcriptase in the presence of either Cy3- or Cy5- labeled dCTP as described (website: http: //www. cmgm.stanford.edu/pbrown/protocols.html). The appropriate Cy3- and Cy5- labeled probes were pooled and hybridized to microarrays in a volume of 25 µl under a 22x14 mm glass coverslip for 16 hr. at 65C and washed at a stringency of 0.2XSSC. The microarray contains 24,000 DNA spots representing approximately 10,000 known full-length cDNAs and 14,000 ESTs of clones made available by Research Genetics.

Fluorescent images of hybridized microarrays were obtained using a genepix 4000 microarray scanner (Axon Instruments, Inc). Images were analyzed with scanalyze (M.Eisen; http://www.microarrays.org/software) or with genepix 3.0. Fluorescence ratios were stored in a custom database. Fluorescent ratios were calibrated independently for each array by applying a single scaling factor to all fluorescent ratios from each array; this scaling factor was computed so that the median fluorescence ratio of the measured spots on each array was 1.0. Genes representing good-quality spots for which the fluorescent intensity in each channel was greater than 1.5 times the local background were selected.

### 6. Northern-blotting

Cells were seeded at low density (3x10⁴ cells/cm²) in the presence or absence of doxycycline (1 µg/ml). After 24 hours of induction cells were collected and RNA was extracted following standard procedures. The following probes were used in the northern blot assays: EphB2 (nucleotides 2960 to 3771), EphB3 (nucleotides 3296 to 3708) and ephrin-B1 (nucleotides 2644 to 2897).

### 7. Induction of EphB/ephrin-B activity using recombinant Fc fusion proteins

Recombinant extracellular domains of the EphB2 receptor or ephrin-B1 ligand fused to the Fc fragments of the human immunoglobulins were from R&D systems, Inc. Human immunoglobulins or Fc-recombinant proteins were crosslinked using anti-Fc antibodies at 1:1 molar ratio in cell medium for 2 hours at room temperature. Ls174T cells were seeded at low density on to laminin-coated plates (2 µg/cm²). After 12 hours, crosslinked IgG or Fc-recombinant proteins were added to the medium at a final concentration of 5 µg/ml. 20 minutes after the addition, cells were fixed with 4% PFA/PBS and stained with Hematoxylin/Eosine or with Phalloidine-TRITC using standard procedures.

### 8. Tissue sample preparation and immunohistochemistry

The intestinal tract was dissected as a whole and flushed gently with cold PBS to remove any fecal content. The small intestine and colon were rolled up into a compact circle and fixed for 24 hours in 4% PFA in PBS at 4°C for 24 hours. Alternatively, 2 cm samples of each of the intestinal segments (duodenum, jejunum, ileum and colon) were collected. Intestinal rolles or individual segments were embedded in paraffin and sectioned (4-5 µm).

For detection of EphB proteins and ephrin ligands, antibodies generated against the extracellular portion of each protein were used as follows: goat anti-EphB2 (1:200; R&D systems), goat anti-EphB3 (1:100; R&D systems), goat anti-ephrin-B1 (1:400; R&D systems), goat anti-ephrin-B2 (1:200; R&D systems), goat anti-ephrin-B3 (1:100; R&D systems). Rabbit anti-ephrin-B1 (1:200; Santa Cruz; C-18) is directed against a common epitope in all ephrin-B members and was used for detection of the three ephrin-B isoforms simultaneously (Pan anti-ephrin-B).

Following dewaxing and hydration, sections were pretreated with peroxidase blocking buffer (100 mM Na-phosphate pH 5.8, 30 mM NaN₃, 0.2% H₂O₂) for 20 minutes at room temperature. Antigen retrieval was performed by boiling samples in Na-citrate buffer (10 mM, pH 6.0). For ß-catenin stainings, samples were boiled in Tris/EDTA solution (40 mM/ 1 mM. pH 8.0). After 20 minutes (45 minutes for ß-catenin), the boiling pan was allowed to slowly cool down to room temperature. Incubation of antibodies was performed in 1% BSA in PBS overnight at 4°C. In all cases, the Envision+ kit (DAKO) was used as a secondary reagent. For goat antibodies, a bridge step using rabbit anti-goat antibodies was required. Stainings were developed using either DAB (brown precipitate) or Vector Vip substrate (purple precipitate). Slides were then counterstained with hematoxylin and mounted.

Double Ki67/Fabp-I immunohistochemistry was performed sequentially following the above procedure with a few modifications. Briefly, after developing Ki67 staining with DAB, a peroxidase-blocking step was performed. Next, free antibody binding sites were block with 5% of normal mouse serum. Then, Fabp-I staining was performed and the results were visualized with Vector-Vip substrate.

### RESULTS

### 1. Generation and characterization of inducible dnTCF cell lines

To determine the role of ß-catenin/TCF complexes in established CRC cells, cell lines were constructed carrying doxycycline-inducible expression plasmids encoding N-terminally truncated versions of TCF factors. Such dominant-negative TCF (dnTCF) proteins do not bind ß-catenin and therefore act as potent inhibitors of the endogenous ß-catenin/TCF complexes present in CRC. As the recipient cell line, the CRC cell line Ls174T, which expresses mutant ß-catenin protein, yet is diploid and carries wild-type alleles of p53 and *APC* was initially chosen. Multiple clones were isolated and tested for inducibility of dnTCF4 expression.

Strong nuclear dnTCF4 staining was observed after doxycycline (Dox) induction of positive transfectants. Accumulation of the induced protein could be detected as early as 4 hours after the addition of doxycycline. CRC cell lines such as Ls174T that carry WNT pathway mutations constitutively activate TCF reporters (pTOPFlash). Several clones were selected in which the inducible expression of dnTCF4 completely abrogated this constitutive pTOPFlash activity.

### 2. The genetic program under the transcriptional control of ß-catenin/TCF activity in CRC cells

By DNA array analysis, it was asked which genes were specifically affected in their expression upon the induction of dnTCF4. mRNA was isolated at 11 hours and 23 hours after the initiation of the experiment with or without the addition of doxycycline. cDNA prepared from the uninduced samples was labeled with Cy3, while the induced cDNA samples were labeled with Cy5. At each time point, the uninduced and induced cDNA samples were mixed and hybridized in duplicate to a DNA array consisting of approximately 24,000 cDNA spots representing known genes or EST clusters. Fluorescent images were analysed as detailed in experimental procedures.

A single criterium was applied to the array data set: i.e. a decrease of at least 2.5 fold in both measurements at the 23 hour time point. This defined a small set of 35 entries that were downregulated when we abrogated ß-catenin/TCF activity in Ls174T cells expressing dnTCF-4 (Table I).

For a number of downstream genes defined in the Ls174T cells expressing dnTCF4, northern blot analysis was performed before and after induction of dnTCF4. This invariably confirmed the DNA array data

To further investigate the effects of ß-catenin/TCF inhibition, Ls174T cells expressing dnTCF1, the natural dominant-negative isoform of TCF1 expressed in the intestinal epithelium were constructed.

Again, doxycycline-induced expression of dnTCFs in all cell lines resulted in the abrogation of pTOPflash activity. Northern blot analysis was performed in the above dnTCF expressing cell lines. Almost invariably, the target genes listed in Table I were also strongly downregulated by dnTCF1 in Ls174T.

### 3. The genetic program controlled by ß-catenin/TCF in CRC cells is physiologically active in colonic epithelium

In order to validate the ß-catenin/TCF target genes described in this example, immunohistochemical analyses of those entries for which antibodies were available were performed on early intestinal neoplastic lesions.

It was found that EphB2 was not only expressed in polyps, but also in cells within the proliferative compartment at the bottom of normal colon crypts. This pattern was invariably confirmed for all target genes tested by immunohistochemistry. These included *c-MYB, BMP4, ENC1, EphB3,* and CD44.

### 4. The EphB2 and EphB3 receptor tyrosine kinases and their ligand ephrin-B1 are inversely controlled by ß-catenin/TCF signaling in intestinal epithelial cells

Among the 30 cDNAs whose levels rapidly dropped upon inhibition of the ß-catenin/TCF-mediated transcription, the EphB2 and EphB3 receptor tyrosine kinases (RTKs) were noticed. Furthermore, their ligand ephrin-B1 was among the 100 genes that were upregulated upon inhibition of ß-catenin/TCF. These data were confirmed by Northern blot analysis. In accordance with the results obtained in the DNA array experiment, the levels of the mRNAs coding for both receptors were dramatically downregulated after 24 hours of induction with doxycycline, while a reverse pattern was seen for ephrin-B1.

The behavior of the ephrin-B1 and -B2 positive cells was studied using an antibody raised against an epitope conserved in all ephrin-B ligands. Ephrin-B expression is very low at positions near the putative stem cell and increases gradually towards the crypt-villus junction. This demonstrates that the amount of Ephrin-B expression can be used to establish the degree of differentiation of a cell. Stem cells have a low expression of Ephrin-B.

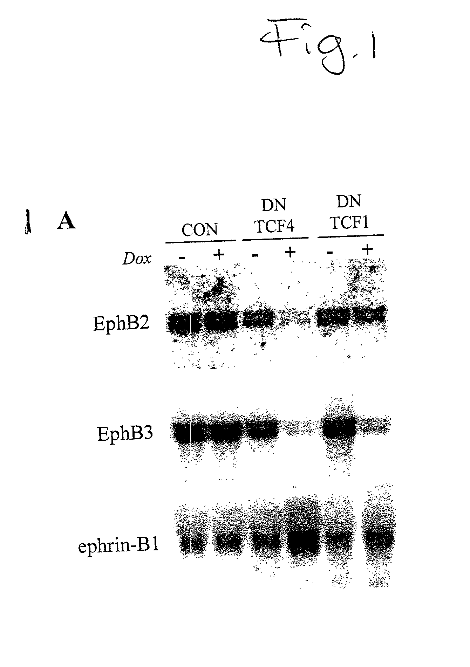

Figure 1 shows that EphB2 and EphB3 are ß-catenin/TCF targets in intestinal cells by the Northern blot analysis of the expression levels of EphB2, EphB3 and ephrin-B1 mRNAs in Ls174T colorectal cancer cells. Cells were induced for 24 hours with doxycycline (dox; 1 µg/ml). CON indicates Ls174T cells transfected with the Tet-repressor expression vector alone.

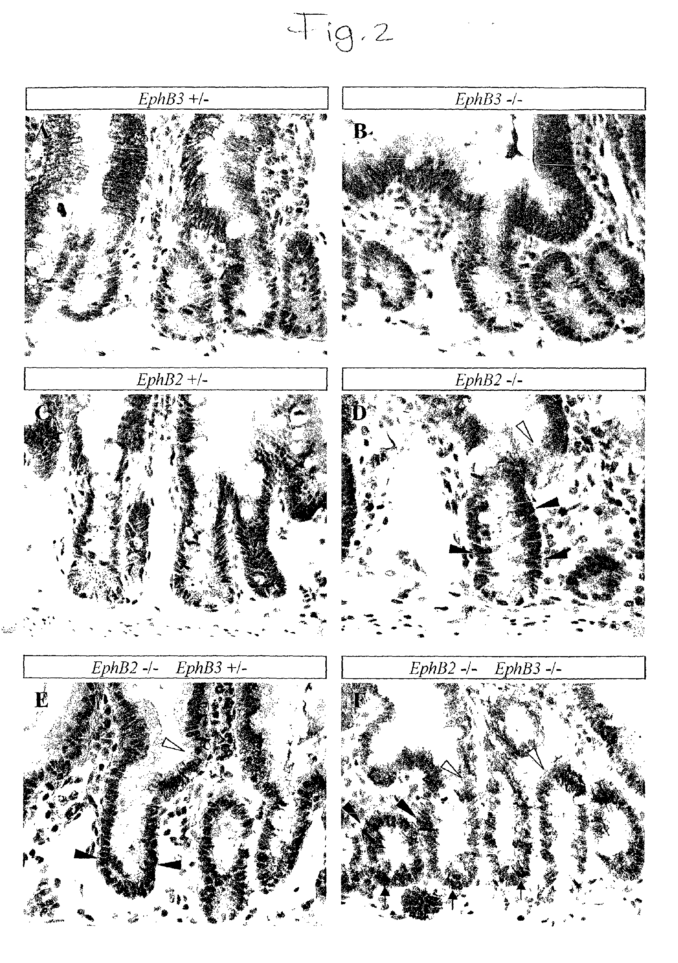

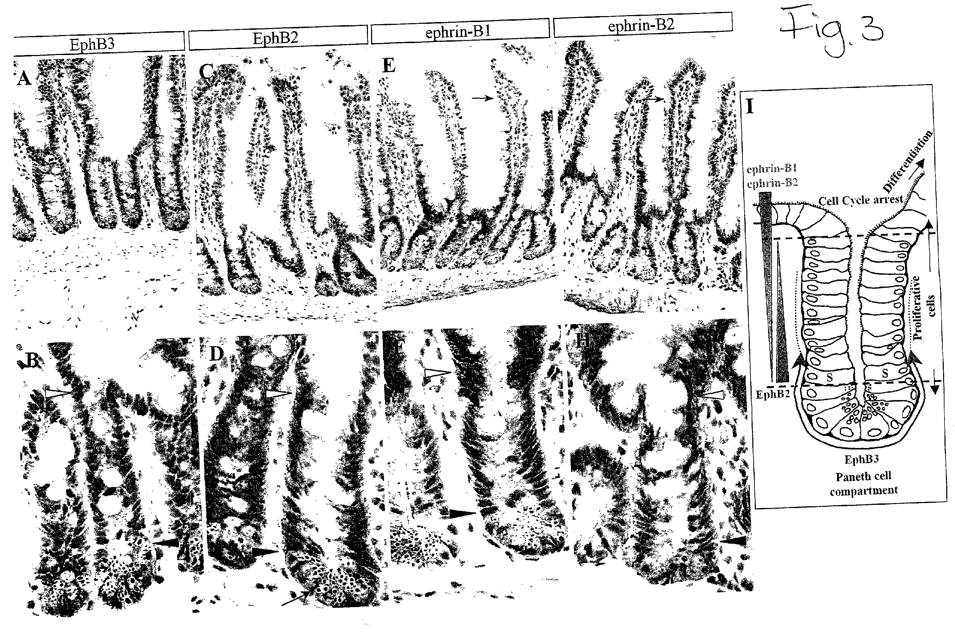

The behavior of the ephrin-B1 and -B2 positive cells using an antibody raised against an epitope conserved in all ephrin-B ligands was studied (Figure 2). Ephrin-B expression is very low at positions near the putative stem cell and increases gradually towards the crypt-villus junction (Figures 3F and 3H; Figures 2A-2C).

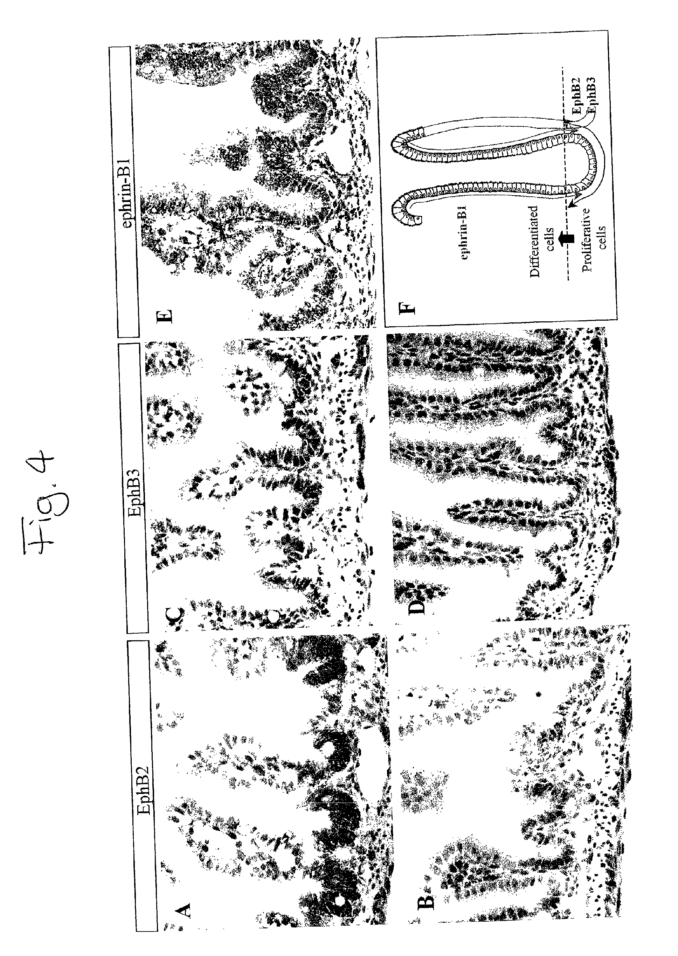

Figure 4 shows the expression pattern of EpHB2, EpHB3 and ephrin-B1 in the small intestine of neonatal mice. Figures 4(A-E) show the immunohistochemical analysis of EpHB2 (A and B), EpHB3 © and D) and ephrin-B1 (E) in the small intestine of newborn animals. Stainings were performed on sections from *EpHB2*^{+/-} (A and E) and *EpHB3*^{+/-} (C) mice. Antibody specificity is demonstrated by the lack of staining in equivalent sections from EpHB2 (B) or EpHB3 (D) homozygous null littermates. Figure 4(E) is a schematic representation of the expression domains of the EpHB2, EpHB3 and their ligand ephrin-B1 in neonatal small intestine. EpHB2 and EpHB3 expression is restricted to the cells in the intervillus regions while ephrin-B1 is expressed in a complementary pattern by the adjacent cells at the villus.

Figure 3 shows the expression pattern of EpHB2, EpHB3, ephrin-B1 and ephrin-B2 in the adult small intestine. Figure 3(A-H) relate to the immunodetection of EpHB2, EpHB3, ephrin-B1 and ephrin-B2 proteins in sections from the jejunum of adult (>7 weeks) mice. The arrow in panel D points to an EpHB2 positive crypt base columnar cell. Black arrowheads indicate the boundary between the Paneth cell compartment and the proliferative compartment. White arrowheads point to the crypt-villus junction. Note that Paneth cells are easily recognizable at the bottom of the crypts by the presence of bright apical granules. Arrows in panels E and G indicate low levels of ephrin-B ligands in cells at the top of the villus. Figure 3(I) is a schematic representation of the expression gradients of EpHB2, EpHB3 and their ephrin ligands in the small intestinal crypts. Arrows show the direction of the migration flow. S indicates the putative stem cell position.

### DISCUSSION

This example describes an analysis of the genetic program controlled by ß-catenin/TCF signaling. To this end, dominant-negative TCF factors were inducibly expressed in colorectal cancer cells. A detailed analysis of the changes in the expression profiles was determined by DNA array technology. As a common denominator, it was found that genes downregulated upon inhibition of ß-catenin/TCF signaling were physiologically expressed in the crypts. In contrast, genes that were upregulated by ß-catenin/TCF inhibition were physiologically expressed in the differentiated compartments of the intestinal epithelium.

### EXAMPLE 2

### Isolation of intestinal stem cells using EphB2, EphB3, CD44 and Ephrin B1 as markers

Intestinal stem cells were isolated by means of monoclonal antibodies directed against human EpHB2, EpHB3, CD44 or Ephrin B1.

A single cell suspension of intestinal tissue was prepared by adapting the method disclosed in Booth, C et al., (1999) Exp. Cell Res. 249: 359). Cultures of crypt cell preparations were supplemented with Wnt conditioned media to maintain the pluripotentency of the stem cells.

Subsequently, the various antibodies and the single cell suspensions are contacted.

Cells binding to the antibodies are identified by means of a FACS cell sorter. Stem cells coexpress high levels of CD44 and EpHB2 and low levels of EpHB3. Additionally, this sorted stem cell population exhibits only low levels EFNB1 expression. High amounts in this case are approximated as being values above 500 under on the log fluorescence scale and low means a value below 200 on the same scale.

## Claims

1. Use of the expression products of the TCF target genes listed in Table 1 as markers for the isolation of intestinal stem cells.

2. Method for the isolation of intestinal stem cells by the following steps:
a) providing antibodies, antibody derivatives or ligand fusion proteins against one or more of the target gene products of the genes listed in Table 2;
b) providing single cell suspensions of intestinal tissue;
c) contacting the antibodies, antibody derivatives or ligand fusion proteins and the single cell suspensions;
d) identifying the cells binding to the antibodies, antibody derivatives or ligand fusion proteins; and
e) optionally isolating the stem cells from the antibodies, antibody derivatives or ligand fusion proteins.

3. Method as claimed in claim 2, wherein at least 2, preferably at least 3, more preferably at least 4 different target gene products are used as markers.

4. Method as claimed in claims 2 and 3, wherein a FACS cell sorter is used to identify and sort the cells that bind the antibody, derivatives thereof or ligand fusion proteins.

5. Method as claimed in claim 4, wherein the stem cells are sorted based on the fact that they coexpress high levels of CD44 and EpHB2 and low levels of EpHB3, and that after sorting the sorted stem cell populations demonstrate only low level EFNB1 expression.

6. Intestinal stem cell preparation obtainable by a method as claimed in claims 2-5.

7. Intestinal stem cell preparation as claimed in claim 6 for use in the manufacture of a therapeutical agent for treating damage to intestinal epithelium.

8. Intestinal stem cell preparation as claimed in claim 6 for use in the manufacture of a therapeutical agent for treating damage to the liver.

9. Intestinal stem cell preparation as claimed in claim 6 for use in the manufacture of a therapeutical agent for treating damage to the pancreas.

10. Therapeutical preparation for treating damage to intestinal epithelium, pancreas or liver, comprising intestinal stem cells as claimed in claim 6.
